# FASCICULE DE BREVET EUROPEEN

(11) **EP 1 992 382 B1**
(45) Date de publication et mention de la délivrance du brevet: **15.02.2012**
(21) Numéro de dépôt: 08305116.9
(22) Date de dépôt: 22.04.2008
(51) Int. Cl.: A61M 16/10, B01D 53/04, F25J 1/00, F17C 13/08, F25J 3/04

(54) **Dispositif de fourniture d'oxygène domestique et ambulatoire**
Vorrichtung zur ortsfesten und ambulanten Sauerstoffversorgung
Device for supplying domestic and ambulatory oxygen

(30) Priorité: 14.05.2007 FR 0755058
(43) Date de publication de la demande: 19.11.2008
(73) Titulaire: L'AIR LIQUIDE, Société Anonyme pour l'Etude et l'Exploitation des Procédés Georges Claude, 75007 Paris (FR)
(72) Inventeur: Sermet, Eric, 77700 Serris (FR); Ferre, Eliette, 75011 Paris (FR); Dodier, Philippe, 69110 Saint Foy Les Lyon (FR); Mazoyer, Joseph, 69110 Saint Foy Les Lyon (FR)
(74) Mandataire: De Cuenca, Emmanuel Jaime

(56) Documents cités:
- WO-A-01/33135
- WO-A-99/11989
- WO-A-2006/047710
- WO-A-2007/118054
- US-B1- 6 346 139
- US-B1- 6 446 630

## Description

La présente invention concerne dispositif de fourniture d'oxygène domestique et ambulatoire.

De nombreuses solutions existent pour proposer de l'oxygène gazeux dans le cadre d'un traitement d'oxygénothérapie. Une solution connue consiste à prévoir une réserve d'oxygène liquide chez le patient à laquelle il est raccordé lorsqu'il est statique chez lui, la réserve lui permettant de remplir un réservoir portable que le patient transporte lors de ses déplacements. Cette solution pose cependant des problèmes de logistique et de distribution pour approvisionner la réserve en oxygène liquide.

Pour s'affranchir de cette distribution d'oxygène liquide, une autre solution consiste à produire de l'oxygène gazeux sous pression à domicile à partir d'un concentrateur. Cette solution nécessite cependant de compresser l'oxygène à une pression élevée, ce qui augmente les risques pour l'utilisateur. Par ailleurs, ce système a en général une faible autonomie et est peu propice par son poids à la déambulation.

Une autre solution propose l'utilisation d'un concentrateur portatif. Cependant, ce type de système dépend alors d'une source d'énergie (batterie) et a un fonctionnement peu satisfaisant du fait notamment : de sa faible robustesse, de son fonctionnement bruyant et de son poids important.

Une autre solution envisagée consiste à produire de l'oxygène liquide au domicile du patient au moyen d'un concentrateur couplé à un liquéfacteur. Cependant les solutions de ce type sont en général très complexes et ergonomiquement peu satisfaisantes (connexions et déconnexions multiples nécessaires suivant le type d'utilisation).

Le document WO 99/11989 décrit par exemple un dispositif de fourniture d'oxygène fixe comprenant un concentrateur, un liquéfacteur et une réserve transportable, le patient étant connecté au liquéfacteur à son domicile mais se connecte à la réserve transportable lors d'un déplacement.

Un but de la présente invention est de pallier tout ou partie des inconvénients de l'art antérieur relevés ci-dessus.

A cette fin, le dispositif de fourniture d'oxygène domestique et ambulatoire selon l'invention est essentiellement caractérisé en ce qu'il comprend une station de remplissage en oxygène et une réserve d'oxygène transportable sélectivement connectable à la station de remplissage, la station de remplissage comprenant un concentrateur d'oxygène destiné à isoler l'oxygène gazeux de l'air, un liquéfacteur connecté à une sortie du concentrateur pour recevoir l'oxygène gazeux isolé en vue de sa liquéfaction, un raccord de transfert relié à une sortie du liquéfacteur et destiné à être connecté à un raccord d'entrée de la réserve, la réserve comprenant un réservoir relié au raccord d'entrée en vue du transfert d'oxygène liquide depuis la station de remplissage vers le réservoir, un système de délivrance comprenant des organes de prélèvement, de réchauffage et de régulation en débit de l'oxygène du réservoir en vue de la délivrance d'oxygène gazeux à un patient, le dispositif de fourniture d'oxygène comprenant un raccord de sortie d'oxygène gazeux destiné à être connecté aux voies respiratoires d'un patient, ledit raccord de sortie d'oxygène étant situé sur la réserve d'oxygène transportable, en aval du système de délivrance, de sorte que le patient peut rester connecté en permanence à la réserve et alimenté en oxygène gazeux par cette dernière, c'est-à-dire aussi bien lorsque la réserve est ou n'est pas raccordée à la station de remplissage.

Le dispositif selon l'invention comporte un unique raccord de sortie d'oxygène gazeux destiné à être connecté aux voies respiratoires d'un patient, ledit raccord unique étant situé sur la réserve d'oxygène transportable, de sorte que le patient doit rester connecté en permanence à la réserve pour être alimenté en oxygène gazeux aussi bien lorsque la réserve est ou n'est pas raccordée à la station de remplissage. Par ailleurs, des modes de réalisation de l'invention peuvent comporter l'une ou plusieurs des caractéristiques suivantes :
- le dispositif comporte un organe de mesure de niveau de liquide dans le réservoir de la réserve, ledit organe de mesure de niveau étant relié à la station de remplissage pour former une boucle de régulation automatique du transfert d'oxygène liquide depuis la station de remplissage vers le réservoir,
- le réservoir de la réserve comprend une sortie pour de l'oxygène gazeux reliée au raccord de sortie via un réchauffeur de gaz et un élément de régulation du flux tel qu'une vanne ou une vanne à régulation de débit,
- le dispositif comporte un élément économiseur apte à assurer le recyclage de l'oxygène gazeux d'ébullition du réservoir vers un patient et un organe de sécurité tel qu'une soupape conformée pour libérer vers l'extérieur de l'oxygène gazeux d'ébullition lorsque la pression au sein du réservoir excède un seuil déterminé et que l'économiseur ne recycle pas ou pas suffisamment d'oxygène gazeux vers le patient,
- l'élément économiseur et l'organe de sécurité sont disposés entre le réchauffeur et l'élément de régulation du flux,
- le réservoir de la réserve comprend une sortie pour de l'oxygène liquide reliée au raccord de sortie via un réchauffeur de liquide et un élément de régulation du flux tel qu'une vanne ou une vanne à régulation de débit,
- le réservoir de la réserve comprend un sortie d'évent pour de l'oxygène gazeux apte à être reliée à la station de remplissage pour un éventuel échange thermique avec l'oxygène gazeux isolé en vue de sa liquéfaction,
- la sortie d'évent est reliée au liquéfacteur pour permettre la mise en froid d'un doigt de liquéfaction du liquéfacteur,
- la station de remplissage comprend un carter repliable et transportable apte à être reliée à une source d'énergie électrique.

D'autres particularités et avantages apparaîtront à la lecture de la description ci-après, faite en référence aux figures dans lesquelles :
- la figure 1 représente une vue schématique illustrant un exemple de dispositif de fourniture d'oxygène domestique et ambulatoire selon l'invention lors d'une utilisation dite « fixe » par un patient (par exemple de nuit),
- la figure 2 représente une vue schématique et partielle du dispositif de fourniture d'oxygène domestique et ambulatoire de la figure 1 lors d'une utilisation dite « déambulatoire » par un patient (par exemple de jour),
- la figure 3 représente une vue en schématique et partielle d'une partie du dispositif des figures 1 et 2 dans une position de manutention (repliement et transport de la base de production d'oxygène du dispositif),
- la figure 4 représente une vue schématique illustrant la structure et le fonctionnement du dispositif de fourniture d'oxygène domestique et ambulatoire selon l'invention lors d'une utilisation fixe,
- la figure 5 représente une vue schématique illustrant la structure et le fonctionnement d'une partie du dispositif de la figure 4 lors d'une utilisation déambulatoire.

En se référant à présent aux figures, le dispositif de fourniture d'oxygène domestique et ambulatoire selon l'invention comprend une station 1 de remplissage en oxygène et une réserve 2 d'oxygène transportable.

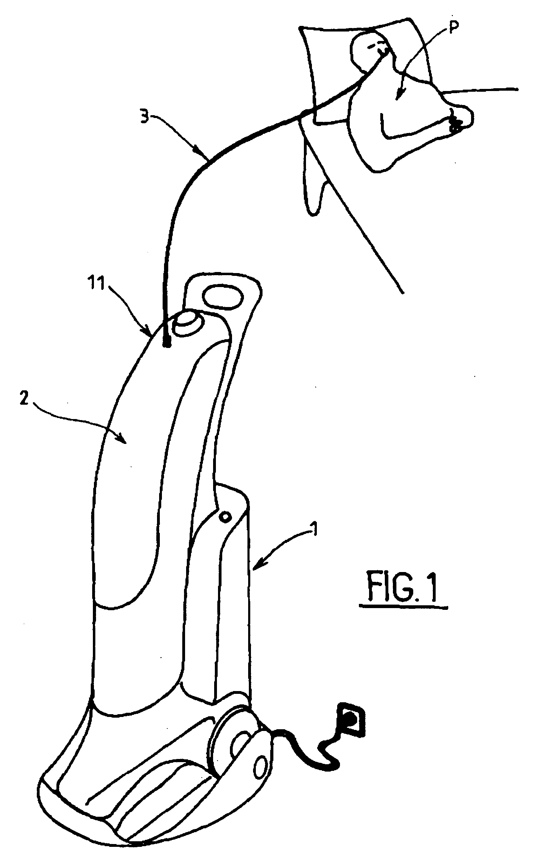

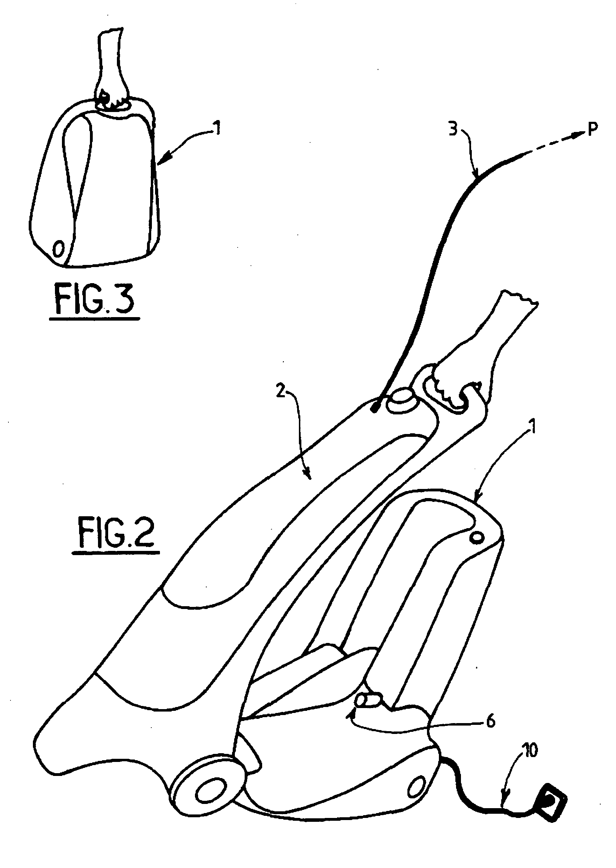

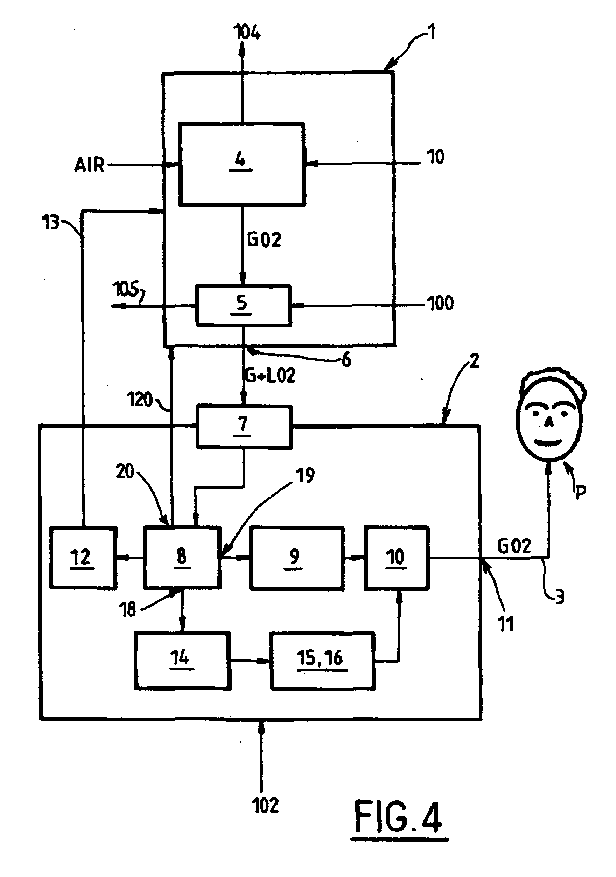

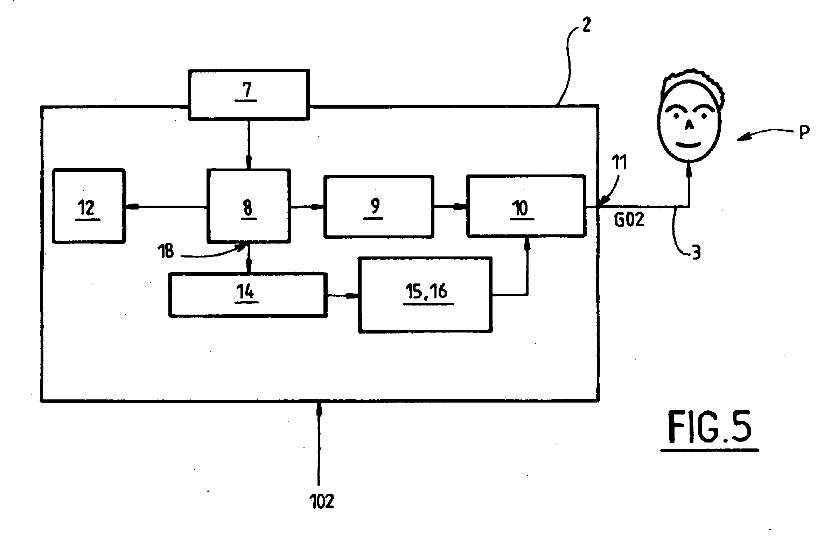

La réserve 2 d'oxygène transportable peut être sélectivement connectée (figures 1 et 4) à la station de remplissage 1 ou déconnectée de la station 1 de remplissage (figures 2 et 5).

En se référant à présent plus précisément à la figure 4, la station 1 de remplissage comprend un concentrateur 4 d'oxygène destiné à isoler l'oxygène gazeux de l'air et un liquéfacteur 5 connecté à une sortie du concentrateur 4 pour recevoir l'oxygène gazeux G02 isolé en vue de sa liquéfaction.

Le concentrateur 4 d'oxygène peut comprendre un système du type à adsorption par oscillation de pression (PSA) ou équivalent.

Classiquement, et comme symbolisé à la figure 4, le concentrateur 4 d'oxygène est alimenté en air et en énergie (telle que de l'énergie électrique d'un réseau) et produit de la chaleur 104.

Le liquéfacteur 5, par exemple du type refroidisseur STIRLING ou un refroidisseur à tube à gaz pulsé est alimenté en gaz enrichi en oxygène par le concentrateur G02, en énergie 100 (par exemple réseau électrique) et génère de la chaleur 105 lors de la liquéfaction de l'oxygène.

Le liquéfacteur 5 produit un mélange d'oxygène liquide et gazeux G+L 02

En sortie, la station 1 de remplissage comprend un raccord de transfert 6 relié la sortie d'oxygène du liquéfacteur 5 (figures 2 et 4) et destiné à être connecté à un raccord d'entrée 7 de la réserve 2.

La réserve 2 comprend un réservoir 8 cryogénique relié au raccord d'entrée 7 en vue du transfert d'oxygène liquide depuis la station 1 de remplissage vers l'intérieur du réservoir 8.

Classiquement, la réserve 2 comprend un système 9, 10 de délivrance d'oxygène gazeux comprenant des organes de prélèvement, de réchauffage et de régulation en débit de l'oxygène du réservoir 8 en vue de son administration à un patient P.

Classiquement, le système de délivrance peut comprendre un organe réchauffeur 9 de liquide (par exemple serpentin échangeur de chaleur avec l'air l'extérieur 102) et des organes 10 de débit (tel qu'au moins une vanne de préférence à débit variable et réglable).

Pour alimenter un patient P en oxygène gazeux la réserve 2 comporte un raccord 11 de sortie d'oxygène gazeux destiné à être connecté 3 (via par exemple une lunette ou un masque) aux voies respiratoires d'un patient P.

Selon une particularité particulièrement avantageuse le patient P peut rester connecté en permanence à la réserve 2 et être alimenté en oxygène gazeux par cette dernière, c'est-à-dire aussi bien lorsque la réserve 2 est raccordée à la station 1 de remplissage que lorsque la réserve 2 n'est pas raccordée à la station 1 de remplissage.

A cet effet, le dispositif peut comporter un unique raccord 11 de sortie d'oxygène gazeux situé sur la réserve 2 d'oxygène transportable de sorte que le patient P doit rester connecter en permanence à la réserve 2 pour être alimenté en oxygène gazeux (de jour : figures 2 et 5, comme de nuit : figures 1 et 4).

De préférence, le dispositif comporte un capteur 12 de mesure de niveau de liquide dans le réservoir 8 de la réserve 2. L'information de mesure de ce capteur 12 de niveau est transmise à la station 1 de remplissage pour former une boucle 13 de régulation automatique du transfert d'oxygène liquide depuis la station 1 de remplissage vers le réservoir 8. C'est-à-dire, que lorsque la réserve 2 est connectée à la station de remplissage 1, en fonction de la mesure du niveau de liquide dans le réservoir 8, la station alimente ou non en oxygène liquide la réserve suivant que le niveau dans le réservoir 8 est inférieur ou supérieur à un seuil.

Par ailleurs, le réservoir 8 de la réserve 2 comprend un sortie 20 d'évent pour de l'oxygène gazeux. Selon une particularité avantageuse, une conduite 120 permet de mettre en échange thermique ce gaz d'évent froid avec l'oxygène gazeux isolé dans la station 1 de remplissage en vue de sa liquéfaction.

Par exemple le gaz d'évent peut être amené par la conduite 120 au liquéfacteur 5 pour permettre par exemple la mise en froid d'un doigt réfrigération du liquéfacteur 5.

Le réservoir 8 de la réserve 2 comprend par ailleurs une sortie 18 pour de l'oxygène gazeux (par exemple l'oxygène d'ébullition ou « boil-off ») reliée au raccord 11 de sortie via un réchauffeur 14 de gaz et un élément 10 de régulation du flux tel qu'au moins une vanne ou une vanne à régulation de débit.

Classiquement, le réchauffeur 14 de gaz peut comprendre un échangeur de chaleur tel qu'un serpentin en échange thermique avec l'air extérieur 102.

Un élément économiseur 15 apte à assurer le recyclage de l'oxygène gazeux d'ébullition du réservoir 8 vers le patient est disposé en aval du réchauffeur 14 de gaz.

L'économiseur 15 assure une régulation automatique de la pression au sein du réservoir 8 en dirigeant le gaz en excès du réservoir 8 vers le patient P (via l'élément 10 de régulation). Lorsque le patient P n'est pas connecté (élément 10 de régulation fermé) et/ou en cas de surpression dangereuse, le gaz est évacué par un organe 16 de sécurité tel qu'une soupape décrite plus en détail ci-dessous.

En amont ou en aval de l'économiseur 15 il est prévu un organe de sécurité 16 tel qu'une soupape conformée pour libérer vers l'extérieur de l'oxygène gazeux d'ébullition lorsque la pression au sein du réservoir 8 et/ou du circuit excède un seuil déterminé.

Ainsi, l'oxygène gazeux en excès dans le réservoir 8 est recyclé vers le patient P au lieu d'être perdu dans l'atmosphère. La consommation d'oxygène du système est ainsi mieux maîtrisée.

Comme représenté aux figures 1 à 3, de préférence la station 1 de remplissage comprend un socle repliable en vue de sa manutention (figure 3).

De même, la réserve 2 peut être intégrée dans un carter monté sur roulettes et comprenant une poignée supérieure de préhension.

L'invention présente de nombreux avantages.

Le raccord 11 unique de sortie d'oxygène permet d'utiliser un unique réservoir 8 d'oxygène liquide, une unique vanne de réglage de débit délivré au patient ... (idem pour les autres organes de régulation de fluide du circuit en sortie de réservoir).

De plus, selon l'invention, le patient est toujours alimenté en oxygène gazeux provenant de l'oxygène liquide de l'unique réservoir 8.

Ceci assure une meilleure pureté de l'oxygène délivré (par rapport à une alimentation directement à la sortie d'un concentrateur gazeux).

## Revendications

1. Dispositif de fourniture d'oxygène domestique et ambulatoire comprenant une station (1) de remplissage en oxygène et une réserve (2) d'oxygène transportable sélectivement connectable à la station de remplissage (1), la station (1) de remplissage comprenant un concentrateur (4) d'oxygène destiné à isoler l'oxygène gazeux de l'air, un liquéfacteur (5) connecté à une sortie du concentrateur (4) pour recevoir l'oxygène gazeux isolé en vue de sa liquéfaction, un raccord de transfert (6) relié à une sortie du liquéfacteur (5) et destiné à être connecté à un raccord d'entrée (7) de la réserve (2), la réserve (2) comprenant un réservoir (8) relié au raccord d'entrée (7) en vue du transfert d'oxygène liquide depuis la station (1) de remplissage vers le réservoir (8), un système (9, 10) de délivrance comprenant des organes de prélèvement, de réchauffage et de régulation en débit de l'oxygène du réservoir (8) en vue de la délivrance d'oxygène gazeux à un patient (P), le dispositif de fourniture d'oxygène comprenant un raccord (11) de sortie d'oxygène gazeux destiné à être connecté (3) aux voies respiratoires d'un patient (P), ledit raccord (11) de sortie d'oxygène étant situé sur la réserve (2) d'oxygène transportable, en aval du système (9, 10) de délivrance, **caractérisé en ce qu'**il comporte un unique raccord (11) de sortie d'oxygène gazeux destiné à être connecté (3) aux voies respiratoires d'un patient (P), ledit raccord (11) unique étant situé sur la réserve (2) d'oxygène transportable, de sorte que le patient (P) doit rester connecté en permanence à la réserve (2) pour être alimenté en oxygène gazeux par cette dernière à partir d'oxygène liquide prélevé dans le réservoir (8), réchauffé et régulé aussi bien lorsque la réserve (2) est ou n'est pas raccordée à la station (1) de remplissage.

2. Dispositif selon la revendication 1, **caractérisé en ce que** le réservoir (8) de la réserve (2) constitue l'unique réservoir de stockage d'oxygène liquide produit par le liquéfacteur (5) du dispositif, de sorte que le patient est alimenté en oxygène à partir d'oxygène liquide produit par le liquéfacteur (5) et stocké uniquement dans l'unique réservoir (8) de la réserve, via l'unique raccord (11) aussi bien lorsque la réserve (2) est ou n'est pas raccordée à la station (1) de remplissage.

3. Dispositif selon la revendication 1 ou 2, **caractérisé en ce qu'**il comporte un organe (12) de mesure de niveau de liquide dans le réservoir (8) de la réserve (2), ledit organe (12) de mesure de niveau étant relié à la station (1) de remplissage pour former une boucle (13) de régulation automatique du transfert d'oxygène liquide depuis la station (1) de remplissage vers le réservoir (8).

4. Dispositif selon l'une quelconque des revendications 1 à 3, **caractérisé en ce que** le réservoir (8) de la réserve (2) comprend une sortie (18) pour de l'oxygène gazeux reliée au raccord (11) de sortie via un réchauffeur (14) de gaz et un élément (10) de régulation du flux tel qu'une vanne ou une vanne à régulation de débit.

5. Dispositif selon la revendication 4, **caractérisé en ce qu'**il comporte un élément économiseur (15) apte à assurer le recyclage de l'oxygène gazeux d'ébullition du réservoir (8) vers un patient (P) et un organe de sécurité (16) tel qu'une soupape conformée pour libérer vers l'extérieur de l'oxygène gazeux d'ébullition lorsque la pression au sein du réservoir (8) excède un seuil déterminé et que l'économiseur ne recycle pas ou pas suffisamment d'oxygène gazeux vers le patient.

6. Dispositif selon la revendication 5, **caractérisé en ce que** l'élément (15) économiseur et l'organe de sécurité (16) sont disposés entre le réchauffeur (14) et l'élément (10) de régulation du flux.

7. Dispositif selon l'une quelconque des revendications 1 à 6, **caractérisé en ce que** le réservoir (8) de la réserve (2) comprend une sortie (19) pour de l'oxygène liquide reliée au raccord (11) de sortie via un réchauffeur (9) de liquide et un élément (10) de régulation du flux tel qu'une vanne ou une vanne à régulation de débit.

8. Dispositif selon l'une quelconque des revendications 1 à 7, **caractérisé en ce que** le réservoir (8) de la réserve (2) comprend un sortie (20) d'évent pour de l'oxygène gazeux apte à être reliée à la station (1) de remplissage pour un éventuel échange thermique avec l'oxygène gazeux isolé en vue de sa liquéfaction.

9. Dispositif selon la revendication 8, **caractérisé en ce que** la sortie (20) d'évent est reliée au liquéfacteur (5) pour permettre la mise en froid d'un doigt de liquéfaction du liquéfacteur (5).

10. Dispositif selon l'une quelconque des revendications 1 à 9, **caractérisé en ce que** la station (1) de remplissage comprend un carter repliable et transportable apte à être reliée à une source (10) d'énergie électrique.

## Claims

1. Device for supplying domiciliary and ambulatory oxygen, comprising an oxygen filling station (1) and a transportable oxygen reserve (2) which can be selectively connected to the filling station (1), the filling station (1) comprising an oxygen concentrator (4) intended to isolate gaseous oxygen from the air, a liquefier (5) connected to an outlet of the concentrator (4) to receive the isolated gaseous oxygen so that it can be liquefied, a transfer connector (6) connected to an outlet of the liquefier (5) and intended to be connected to an inlet connector (7) of the reserve (2), the reserve comprising a tank (8) connected to the inlet connector (7) so that liquid oxygen can be transferred from the filling station (1) to the tank (8), a delivery system (9, 10) comprising members for tapping, heating and regulating the flow of the oxygen from the tank (8) so as to deliver gaseous oxygen to a patient (P), the oxygen supply device comprising a gaseous oxygen outlet connector (11) intended to be connected (3) to the airways of a patient (P), said oxygen outlet connector (11) being situated on the transportable oxygen reserve (2) downstream of the delivery system (9, 10), **characterised in that** it comprises a single gaseous oxygen outlet connector (11) intended to be connected (3) to the airways of a patient (P), said single connector (11) being situated on the transportable oxygen reserve (2) such that the patient (P) has to remain constantly connected to the reserve (2) in order to be supplied with gaseous oxygen by said reserve, from liquid oxygen which is tapped from the tank (8), heated and regulated, both when the reserve (2) is connected to the filling station (1) and when it is not.

2. Device according to claim 1, **characterised in that** the tank (8) of the reserve (2) constitutes the only storage tank for liquid oxygen produced by the liquefier (5) of the device, such that the patient is supplied with oxygen, from liquid oxygen produced by the liquefier (5) and stored only in the single tank (8) of the reserve, via the single connector (11) both when the reserve (2) is connected to the filling station (1) and when it is not.

3. Device according to either claim 1 or claim 2, **characterised in that** it comprises a member (12) for measuring the level of liquid in the tank (8) of the reserve (2), said level measurement member (12) being connected to the filling station (1) to form an automatic control loop (13) for controlling the transfer of liquid oxygen from the filling station (1) to the tank (8).

4. Device according to any one of claims 1 to 3, **characterised in that** the tank (8) of the reserve (2) comprises a gaseous oxygen outlet (18) connected to the outlet connector (11) via a gas heater (14) and a flow regulating element (10) such as a valve or a flow regulating valve.

5. Device according to claim 4, **characterised in that** it comprises an economiser element (15) capable of recirculating the boiled-off gaseous oxygen from the tank (8) to a patient (P) and a safety member (16) such as a relief valve designed to release the boiled-off gaseous oxygen to the outside when the pressure within the tank (8) exceeds a certain threshold and the economiser is not recirculating or not recirculating enough gaseous oxygen to the patient.

6. Device according to claim 5, **characterised in that** the economiser element (15) and the safety member (16) are positioned between the heater (14) and the flow regulating element (10).

7. Device according to any one of claims 1 to 6, **characterised in that** the tank (8) of the reserve (2) comprises a liquid oxygen outlet (19) connected to the outlet connector (11) via a liquid heater (9) and a flow regulating element (10) such as a valve or a flow regulating valve.

8. Device according to any one of claims 1 to 7, **characterised in that** the tank (8) of the reserve (2) comprises a gaseous oxygen vent outlet (20) which can be connected to the filling station (1) for a possible exchange of heat with the isolated gaseous oxygen with a view to liquefying it.

9. Device according to claim 8, **characterised in that** the vent outlet (20) is connected to the liquefier (5) in order to cool a liquefaction tube of the liquefier (5).

10. Device according to any one of claims 1 to 9, **characterised in that** the filling station (1) comprises a foldable, transportable housing which can be connected to a source (10) of electrical power.

## Patentansprüche

1. Vorrichtung für die Zufuhr von häuslichem und ambulantem Sauerstoff, umfassend eine Sauerstoff-Füllstation (1) und einen transportablen Sauerstoffspeicher (2), der selektiv an die Füllstation (1) anschließbar ist, wobei die Füllstation (1) einen Sauerstoff-Konzentrator (4) umfasst, der für die Abscheidung des gasförmigen Sauerstoffs von der Luft vorgesehen ist, einen Verflüssiger (5), der an einen Auslass des Konzentrators (4) angeschlossen ist für die Aufnahme des abgeschiedenen gasförmigen Sauerstoffs für seine Verflüssigung, einen Übertragungsverbinder (6), der mit einem Auslass des Verflüssigers (5) verbunden ist und dafür vorgesehen ist, an einen Einlassverbinder (7) des Speichers (2) angeschlossen zu werden, wobei der Speicher (2) einen Behälter (8) umfasst, der mit dem Einlassverbinder (7) verbunden ist für die Übertragung von flüssigem Sauerstoff von der Füllstation (1) zu dem Behälter (8), ein Abgabesystem (9, 10), das Organe für die Entnahme, die Erhitzung und die Durchflussregelung des Sauerstoffs aus dem Behälter (8) umfasst für die Abgabe von gasförmigem Sauerstoff an einem Patienten (P), wobei die Vorrichtung für die Zufuhr von Sauerstoff einen Verbinder (11) für den Auslass von gasförmigem Sauerstoff umfasst, der für den Anschluss (3) an die Atemwege eines Patienten (P) vorgesehen ist, wobei der Verbinder (11) für den Auslass von Sauerstoff an dem transportablen Sauerstoffspeicher (2), stromabwärts von dem Abgabesystem (9, 10), angeordnet ist, **dadurch gekennzeichnet, dass** sie einen einzigen Verbinder (11) für den Auslass von gasförmigem Sauerstoff aufweist, der für den Anschluss (3) an die Atemwege eines Patienten (P) vorgesehen ist, wobei der einzige Verbinder (11) an dem transportablen Sauerstoffspeicher (2) angeordnet ist, derart, dass der Patient (P) permanent an den Speicher (2) angeschlossen bleiben muss, um durch diesen Letzten mit gasförmigem Sauerstoff aus dem flüssigem Sauerstoff, der aus dem Behälter (8) entnommen, erhitzt und geregelt wird, versorgt zu werden, gleichwohl, wenn der Speicher (2) an die Füllstation (1) angeschlossen oder nicht angeschlossen ist.

2. Vorrichtung nach Anspruch 1, **dadurch gekennzeichnet, dass** der Behälter (8) des Speichers (2) den einzigen Behälter zur Speicherung des flüssigen Sauerstoffs, der von dem Verflüssiger (5) der Vorrichtung erzeugt wird, bildet, derart, dass der Patient mit Sauerstoff aus dem flüssigen Sauerstoff, der durch den Verflüssiger (5) erzeugt und einzig in dem einzigen Behälter (8) des Speichers gespeichert wird, über den einzigen Verbinder (11) versorgt wird, gleichwohl, wenn der Speicher (2) an die Füllstation (1) angeschlossen oder nicht angeschlossen ist.

3. Vorrichtung nach Anspruch 1 oder 2, **dadurch gekennzeichnet, dass** sie ein Organ (12) zur Messung des Füllstands der Flüssigkeit in dem Behälter (8) des Speichers (2) aufweist, wobei das Füllstands-Messorgan mit der Füllstation (1) verbunden ist, um eine Schleife (13) für die automatische Regelung der Übertragung von flüssigem Sauerstoff von der Füllstation (1) zu dem Behälter (8) zu bilden.

4. Vorrichtung nach einem der Ansprüche 1 bis 3, **dadurch gekennzeichnet, dass** der Behälter (8) des Speichers (2) einen Auslass (18) für gasförmigen Sauerstoff umfasst, der mit dem Auslassverbinder (11) über einen Gaserhitzer (14) und ein Element (10) zur Regelung der Durchflussmenge wie ein Ventil oder ein Durchflussregelventil verbunden ist.

5. Vorrichtung nach Anspruch 4, **dadurch gekennzeichnet, dass** sie ein Sparregler-Element (15), das dafür geeignet ist, die Rückführung von siedendem, gasförmigem Sauerstoff von dem Behälter (8) zu einem Patienten (P) sicherzustellen, und ein Sicherheitsorgan (16) wie ein Ventil aufweist, das für die Freisetzung des siedenden, gasförmigen Sauerstoffs nach außen vorgesehen ist, wenn der Druck im Innern des Behälters (8) einen bestimmten Schwellwert übersteigt und wenn der Sparregler den gasförmigen Sauerstoff zu dem Patienten nicht oder nicht ausreichend rückführt.

6. Vorrichtung nach Anspruch 5, **dadurch gekennzeichnet, dass** das Sparregler-Element (15) und das Sicherheitselement (16) zwischen dem Erhitzer (14) und dem Element (10) zur Regelung der Durchflussmenge angeordnet sind.

7. Vorrichtung nach einem der Ansprüche 1 bis 6, **dadurch gekennzeichnet, dass** der Behälter (8) des Speichers (2) einen Auslass (19) für flüssigen Sauerstoff umfasst, der mit dem Auslassverbinder (11) über einen Flüssigkeitserhitzer (9) und ein Element (10) zur Regelung der Durchflussmenge wie ein Ventil oder ein Durchflussregelventil verbunden ist.

8. Vorrichtung nach einem der Ansprüche 1 bis 7, **dadurch gekennzeichnet, dass** der Behälter (8) des Speichers (2) einen Lüftungsauslass (20) für gasförmigen Sauerstoff umfasst, der dafür geeignet ist, mit der Füllstation (1) für einen eventuellen Wärmeaustausch mit dem abgeschiedenen gasförmigen Sauerstoff für seine Verflüssigung verbunden zu werden.

9. Vorrichtung nach Anspruch 8, **dadurch gekennzeichnet, dass** der Lüftungsauslass (20) mit dem Verflüssiger (5) verbunden ist, um die Kühlung eines Verflüssigungsfingers des Verflüssigers (5) zu ermöglichen.

10. Vorrichtung nach einem der Ansprüche 1 bis 9, **dadurch gekennzeichnet, dass** die Füllstation (1) ein klappbares und transportables Gehäuse umfasst, das mit einer elektrischen Energiequelle (10) verbunden werden kann
